(19)

Europäisches
Patentamt
European
Patent Office
Office européen
des brevets

(11) **EP 1 938 847 B1**

(12) **EUROPEAN PATENT SPECIFICATION**

(45) Date of publication and mention
of the grant of the patent:
**11.06.2014 Bulletin 2014/24**

(51) Int Cl.:
***A61M 1/16*** *(2006.01)*   ***A61M 1/34*** *(2006.01)*

(21) Application number: **08001550.6**

(22) Date of filing: **31.10.2002**

(54) **Apparatus for determining access flow**

Vorrichtung zur Bestimmung des Blutzugangsflusses

Appareil pour déterminer le débit d'accès

(84) Designated Contracting States:
**AT BE BG CH CY CZ DE DK EE ES FI FR GB GR
IE IT LI LU MC NL PT SE SK TR**

(30) Priority: **08.02.2002 SE 0200370**

(43) Date of publication of application:
**02.07.2008 Bulletin 2008/27**

(62) Document number(s) of the earlier application(s) in
accordance with Art. 76 EPC:
**02783349.0 / 1 471 957**

(73) Proprietor: **Gambro Lundia AB
220 10 Lund (SE)**

(72) Inventors:
• **Bene, Bernard
69540 Irigny (FR)**
• **Goux, Nicolas
69290 Crapponne (FR)**

• **Hansson, Per
216 16 Limhamn (SE)**
• **Hertz, Thomas
22732 Lund (SE)**
• **Jansson, Olof
23538 Vellinge (SE)**
• **Persson, Roland
21612 Limhamn (SE)**
• **Sternby, Jan
22652 Lund (SE)**
• **Asbrink, Perry
21581 Malmo (SE)**

(74) Representative: **Ponzellini, Gianmarco et al
Ponzellini, Gioia e Associati S.r.l.
Via Mascheroni, 31
20145 Milano (IT)**

(56) References cited:
**EP-A- 0 658 352    EP-A- 0 928 614
WO-A-00/24440**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The present invention relates to a method and apparatus for determining fluid flow rate in a patient's blood access. More particularly, the invention relates to the calculation of the fluid flow rate in the blood access based on conductivity measurements of the post dialyzer or other blood treatment unit effluent fluid.

**BACKGROUND ART**

There are several types of treatments in which blood is taken out in an extracorporeal blood circuit. Such treatments involve, for example, hemodialysis, hemofiltration, hemodiafiltration, plasmapheresis, blood component separation, blood oxygenation, etc. Normally, blood is removed from a blood vessel at a blood access and returned to the same blood vessel.

In hemodialysis and similar treatments, a blood access commonly surgically created in the nature of a arteriovenous shunt, commonly referred to as a fistula. Blood needles are inserted in the fistula. Blood is taken out from the fistula via a needle at an upstream position and blood is returned to the fistula via needle at a downstream position.

The arterio-venous shunt or fistula is blood access having capability of providing a high blood flow and being operative during several years and even tens of years. It is produced by operatively connecting, for example, the radial artery to the cephalic vein at the level of the forearm. The venous limb of the fistula thickens during the course of several months, permitting repeated insertion of dialysis needles.

An alternative blood access to the fistula is the arteriovenous graft, in which a connection is generated from, for example, the radial artery at the wrist to the basilic vein. The connection is made with a tube graft made from e.g. autogenous saphenous vein or from polytetrafluoroethylene (PTFE, Teflon). The needles are inserted in the graft.

A further example of a blood access is a silicon, dual-lumen catheter surgically implanted into one of the large veins.

Further type of blood access find use in specific situations, like a no-needle arterio-venous graft consisting of a T-tube linked to a standard PTFE graft. The T-tube is implanted in the skin. Vascular access is obtained either by unscrewing a plastic plug or by puncturing a septum of said T-tube with a needle. Other methods and devices are also known.

During the above blood treatment therapies, hemodialysis for instance, it is desirable to obtain a constant blood flow rate of 150 - 500 ml/min or even higher, and the access site must be prepared for delivering such flow rates. The blood flow in an AV fistula is often 800 ml/min or larger, permitting delivery of a blood flow rate in the desired range.

In the absence of a sufficient forward blood flow, the extracorporeal circuit blood pump will take up some of the already treated blood entering the fistula via the venous needle, so called access or fistula recirculation, leading to poor treatment results and progressive reduction of treatment efficiency.

A common cause of poor flow with AV fistulas is partial obstruction of the venous limb due to fibrosis secondary to multiple venipunctures. Moreover, stenosis causes a reduction of access flow.

It has been found that access flow rate often exhibit a long plateau time period with sufficient access flow, followed by a short period of a few weeks with markedly reduced access flow leading to recirculation and ultimately access failure. By constantly monitoring the evolution of the access flow during consecutive treatment sessions, it is possible to detect imminent access flow problems. Proper detection of access flow reduction may help in carrying out a maintenance procedure on the access thereby avoiding any access failure.

A non-invasive technique that allows measurement of flow through AV fistulas and grafts is colour Doppler ultrasound. Magnetic Resonance Imaging (MRI) has also been used. However, these techniques require expensive equipment and are not easily used in the dialysis clinic environment.

Several methods have been suggested for monitoring recirculation and access flow. Many of these methods involve injection of a marker substance in blood, and the resultant recirculation is detected. The methods normally involve measurement of a property in the extracorporeal blood circuit. Examples of such methods can be found in US 5,685,989, US 5,595,182, US 5,453,576, US 5,510,716, US 5,510,717, US 5,312,550, etc.

Such methods have the disadvantage that they require the injection of the marker substance and external equipment for the measurements.

More recently, EP 928 614 and WO 00/24440, suggest to measure a post dialyzer concentration of a substance, in particular urea in the effluent fluid before and after a flow reversal, i.e. before the flow reversal the arterial line carries blood from an upstream position of the blood access, and the venous line carries blood towards a downstream position of the blood access, whereas the arterial line carries blood from an downstream position of the blood access, and the venous line carries blood towards a upstream position of the blood access after the flow reversal. A valve for such reversal is shown in i.e. US 5,605,630 and US 5,894,011. A disadvantage in these methods is the requirement for special equipment for measuring the urea concentration. Urea sensors are as such available but they are not standard equipment for most of the dialysis monitors and they have also a considerable maintenance costs.

## SUMMARY OF THE INVENTION

On this background, it is the obj ect of the present invention to provide an apparatus configured for carrying out a method for checking the operating configuration of the arterial and venous lines, as disclosed in any one of the appended claims.

Note that in the present description and in the claims Cn refers always to conductivity-concentration of the effluent dialysis fluid in normal configurations of the lines while Cr refers always to conductivity-concentration of the effluent dialysis fluid in reversed configuration of the lines. If the time sequence adopted is first reversed than normal configuration: the first post treatment unit conductivity-concentration of the dialysis liquid is Cr while the second post treatment unit conductivity-concentration is Cn. If the time sequence adopted is first normal than reversed configuration: the first post treatment unit conductivity-concentration of the dialysis liquid is Cn while the second post treatment unit conductivity-concentration is Cr.

During execution of the above-disclosed method, the post treatment unit conductivities (first and second) are measured after a delay allowing equilibrium to establish.

According to a feature of the invention, the post treatment unit conductivity after the flow reversal is measured at various intervals or continuously so that the value of the conductivity at the time of the flow reversal can be determined by extrapolating the measured values backwards to the moment of the flow reversal. In this way the method can compensate for drift of parameters between the time when the flow is reversed until the time where a substantial equilibrium is reached.

## BRIEF DESCRIPTION OF THE DRAWINGS

In the following detailed portion of the present description, the invention will be explained in more detail with reference to the exemplary embodiments shown in the drawings, in which

Fig. 1 is a partially schematic view of a forearm of a patient provided with an AV fistula.

Fig. 2 is a schematic diagram of an extracorporeal circuit and part of the fluid path of a dialysis machine.

Fig. 3 is a schematic diagram of an extracorporeal circuit including a flow reversal valve.

Fig. 4 is the schematic diagram of Fig. 3, with the valve turned for reversed blood flow

Fig. 5 is a graph showing the conductivities before and after flow reversal, and

Fig. 6 is another graph showing the conductivities before and after flow reversal.

## DESCRIPTION OF DETAILED EMBODIMENTS OF THE INVENTION

For the purpose of this description, a blood access is a site in which a fluid in a tube can be accessed and removed from and/or returned to the tube. The tube may be a blood vessel of a mammal, or any other tube in which a fluid is flowing. The general term blood access as used here includes arterio-venous fistulas, arterio-venous grafts, and dual-lumen catheters amongst other similar types of blood access that allow for an upstream access position and a downstream access position.

The general terms dialyzer or blood treatment unit as used here include filters for hemodialysis, hemofilters, hemodiafilters, plasmafilters and ultrafilters.

The fluid flow rate is the flow rate of the fluid in the tube or blood vessel immediately upstream of the blood access, denoted Qa.

The general term dialysis as used here includes hemodialysis, hemofiltration, hemodiafiltration and therapeutic plasma exchange (TPE), among other similar treatment procedures.

The general term effluent fluid as used here refers to the dialysis fluid downstream of the dialyzer or blood treatment unit.

The general term "transport of substances or ions though the semi permeable membrane" includes any parameter that is indicative of the rate at which substances or ions pass through the dialyzer membrane. Examples of such parameters are, clearance, urea clearance, dialysance, ionic dialysance and effective ionic dialysance.

The general term ionic dialysance as used here refers to a variable that expresses the transport of ions through the dialyzer membrane. The ionic dialysance is ion dependent, i.e. different ions have different dialysance values. It is also dependent on blood flow, dialysate flow and Quf, so during measurements when determining the access flow these must preferably be held constant. The effective ionic dialysance, herein denoted D, further depends on recirculation effects in the fistula and the cardiopulmonary circuit, and is obtained for example as described by EP 658 352. The major ions determining the conductivity of dialysate liquid are sodium and chloride

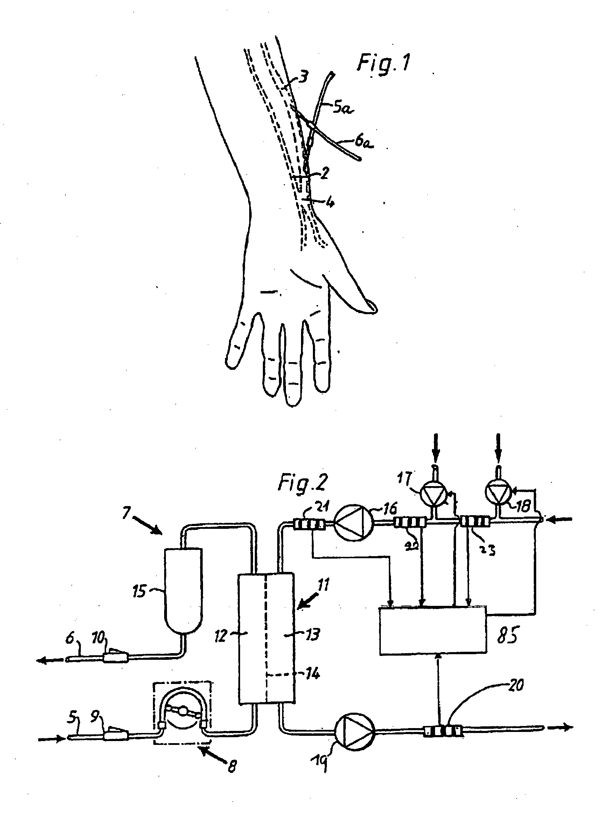

Fig. 1 discloses a forearm 1 of a human patient. The forearm 1 comprises an artery 2, in this case the radial artery, and a vein 3, in this case the cephalic vein. Openings are surgically created in the artery 2 and the vein 3 and the openings are connected to form a fistula 4, in which the arterial blood flow is cross-circuited to the vein. Due to the fistula, the blood flow through the artery and vein is increased and the vein forms a thickened area downstream of the connecting openings. When the fistula has matured after a few months, the vein is thicker and may be punctured repeatedly. Normally, the thickened vein area is called a fistula.

An arterial needle 5a, to which is connected a piece of tube, is placed in an upstream position in the fistula, in the enlarged vein close to the connected openings and a venous needle 6a, to which is connected a piece of tube, is placed in a position downstream of the arterial needle, normally at least five centimeters downstream thereof.

As described above, the blood access can also be an arterio-venous graft, a double lumen catheter or other similar arrangements.

The needles 5a and 6a are connected to a tube system, shown in Fig. 2, forming an extracorporeal circuit 7 comprising a blood pump 8, such as a peristaltic pump. The blood pump propels blood from the fistula, through the arterial needle, the extracorporeal circuit, the venous needle, and back into the fistula.

The extracorporeal blood circuit 7 shown in Fig. 2 further comprises an arterial clamp 9 and a venous clamp 10 for isolating the patient from the extracorporeal circuit should an error occur.

Downstream of pump 8 is a dialyzer 11, comprising a first, so called blood chamber 12 and a second, so called dialysis fluid chamber 13 separated by a semi permeable membrane 14. Further downstream of the dialyzer is a drip chamber 15, separating air from the blood therein.

The bloodline upstream of the dialyzer 11 is referred to as the arterial line 5, whereas the bloodline downstream from the dialyzer 11 is known as the venous line 6. The arterial and venous lines 5 and 6 are able to be configured according to at least a normal configuration, in which said arterial line carries blood from said upstream position of said blood access and said venous line carries blood towards said downstream position of said blood access, and to at least a reversed configuration, in which said arterial line carries blood from said downstream position of said blood access and said venous line carries blood towards said upstream portion of said blood access.

In the normal configuration, blood passes from the arterial needle past the arterial clamp 9 to the blood pump 8. The blood pump drives the blood through the dialyzer 11 and further via the drip chamber 15 and past the venous clamp 10 back to the patient via the venous needle. The drip chamber may comprise an air detector, adapted to trigger an alarm should the blood emitted from the drip chamber comprise air or air bubbles. The blood circuit may comprise further components, such as pressure sensors etc.

The dialysis fluid chamber 14 of the dialyzer 11 is provided with dialysis fluid via a first pump 16, which obtains dialysis fluid from a source of pure water, normally RO-water, mixed with one or several concentrates of ions, varying means including metering pumps 17 and 18 being shown for metering such concentrates. Sensors comprising a conductivity cell 22 and a conductivity cell 23 are provided downstream of the points where the concentrates are mixed into the main fluid steam. The signal of the respective conductivity cell 22,23 is in a closed loop manner compared with the desired conductivity and the speed of the pumps 17 and 18 are controlled in response. A further conductivity cell 21, connected to the protective system of the dialysis machine, is provided downstream from all concentrate mixing steps measuring the final total conductivity. The protective system compares the measured final conductivity with a calculated final conductivity and puts the dialysis machine in a safe state, if anything should have gone wrong in the mixing steps.

A control unit 85 operates said varying means for circulating a dialysis liquid in the second chamber of said treatment unit in such a way that, at least for a time interval T, said dialysis liquid upstream the treatment unit has a concentration (Ci) of one or more substances different from the concentration of the same substance(s) in blood.

According to an embodiment of the invention the difference in concentration is measured as a difference in the conductivity, because most of the components in the dialysis liquid are electrolytes and thus a change in their concentration will inherently lead to a change in the conductivity of the dialysis liquid. It will be understood though, that the invention can also be carried out using the concentration of substances that have no or little effect on the conductivity of the liquid that they are dissolved in, such as urea or glucose.

A preferable range for the dialysate conductivity during the blood access flow measurement is 14,5 to 17,5 mS/cm, preferably about 15 to 16 mS/cm. Thus a conductivity difference between the blood and the dialysate of about 1 to 2 mS/cm is created.

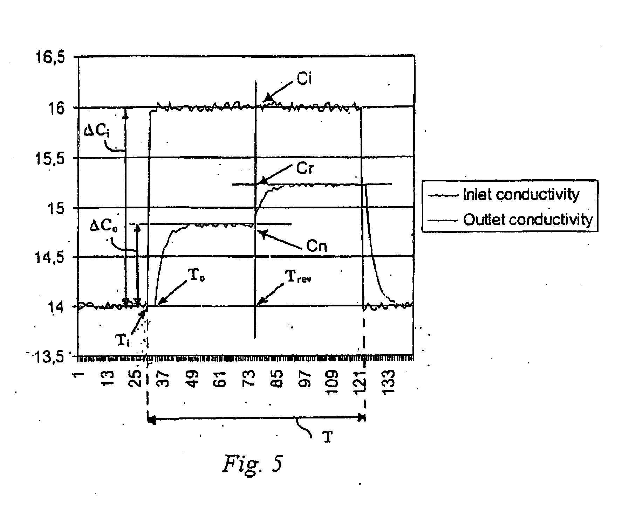

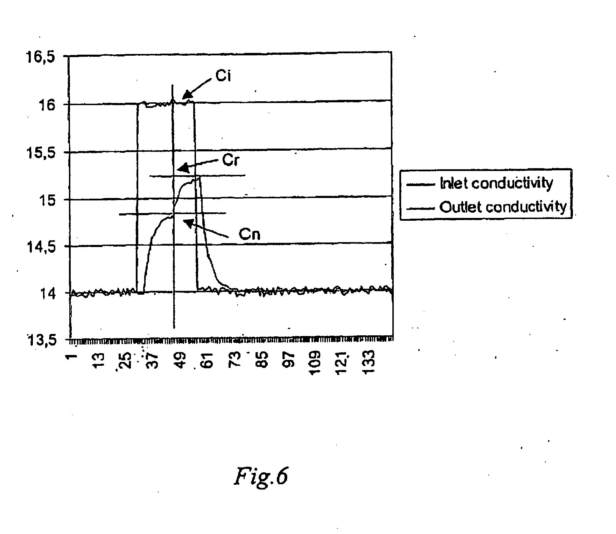

In the specific embodiment shown in figures 5 and 6 an increase in conductivity (concentration of one or more electrolytes) is applied to the fluid upstream the second chamber 13. Said increase starts at time Ti in order to bring the second chamber inlet conductivity to a substantially constant value Ci for a certain time interval T.

According to a first alternative, the invention can work even if instead of an increase a decrease in conductivity or concentration is applied to the fluid at the inlet of the second chamber.

According to a second alternative, if the dialysis liquid inherently has the required difference in conductivity with respect to the blood, then no change in conductivity shall be created for performing the method according to the invention.

A major contribution to the conductivity of the dialysis liquid is sodium chloride. From a physiological standpoint and for best control, the preferred way to adjust the final total conductivity is therefore to change the concentration of sodium chloride. The control unit 85 changes the setting of sodium chloride and in response the speed of metering pump 17 and/or 18 is adjusted as described above. In many types of dialysis apparatus however, the sodium chloride is in a concentrate container together with all the minor amounts of other electrolytes e.g. potassium, magnesium, calcium and peracetic acid, the so called "A concentrate". This concentrate contributes about 12 mS/cm of the usual final 14 mS/cm conductivity. The remainder of the conductivity comes from the bicarbonate concentrate. In such a dialysis machine (not shown) the conductivity is set by changing the amount of A concentrate in the same way as described above for sodium chloride alone.

Though less attractive from a physiological point of view, it is also possible to change the concentration of all electrolytes, i.e. inclusive bicarbonate simultaneously. It is also possible to change the concentration of any other electrolytes or other components such as glucose.

An exchange of substances between the blood and the dialysis fluid takes place in the dialyzer 11 through the semi permeable membrane 14. The exchange may take place by diffusion under the influence of a concentration gradient, so called hemodialysis, and/or by convection due to a flow of liquid from the blood to the dialysis fluid, so called ultrafiltration.

From the dialysis fluid chamber 14 of the dialyzer is emitted a fluid called the effluent fluid, which is driven by a second pump 19 via a conductivity cell 20 to drain. The conductivity cell measures continuously or at various intervals, the conductivity of the effluent fluid emitted from the dialyzer, to provide an effluent fluid conductivity.

As described above, the present invention provides a method of non-invasively measuring the fluid flow in the fistula immediately before the arterial needle, using the conductivity cell 20 and the dialysis circuit as shown in Fig. 2.

By measuring the first post dialyzer liquid conductivity-concentration during normal dialysis (or normal configuration of the venous and arterial lines) and then reversing the positions of the needles (reversed configuration) and measuring the second post dialyzer conductivity-concentration with the needles in the reversed position, the control unit is able to calculate the blood flow in the blood access, without the addition of any substance to the blood or the dialysis fluid solely for the sake of the measurement.

Note that in order to pass from the normal configuration of the lines to the reversed configuration of the lines the following alternative options can be used.

One way of achieving flow reversal in the needles is by manually disconnecting the needles from the bloodlines and reconnecting the arterial needle to the venous bloodline and the venous needle to the arterial bloodline (not shown). Various other ways for achieving the flow reversal are known to the skilled person.

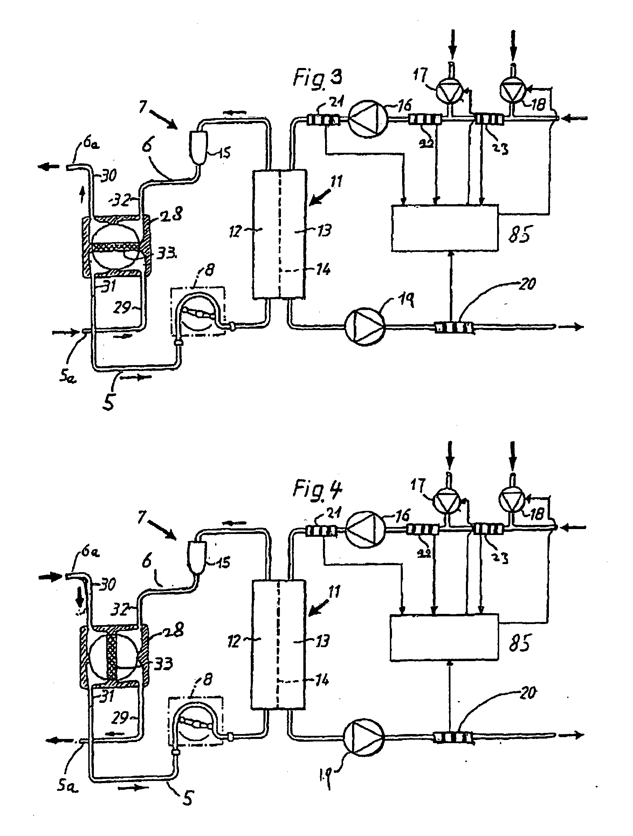

Another embodiment usable for switching the lines between the normal and the reversed condition and vice-versa is shown in Figs. 3 and 4. These figures relate to a schematic diagram of the dialysis circuit according to Fig. 2 with the addition of a valve 28 to perform the flow reversal. The arterial needle 5a is connected to an arterial inlet line 29 of the valve and the venous needle 6a is connected to a venous inlet line 30 of the valve. The blood pump is connected via arterial line 5 to a first outlet line 31 of the valve and the blood returning from the dialyzer 11 is connected via the venous line 6 to a second outlet line 32 of the valve. The valve 28 comprises a valve housing and a pivotable valve member 33, which is pivotable from the normal position shown on the drawing to a reverse position pivoted 90° in relation to the normal position. In the normal position shown in Fig. 3, the arterial needle 5a is connected to the blood pump 8 and the venous needle 6a is connected to the outlet of the dialyzer, via the drip chamber 15. In the reversed position shown in Fig. 4, the arterial needle 5a is connected to the outlet of the dialyzer and the venous needle 6a is connected to the blood pump 8, as required. Thus the flow is "reversed", and the arterial line 5 carries blood from a downstream position of the blood access, and the venous line 6 carries blood towards an upstream position of the blood access. According to an embodiment, the dialysis machine automatically controls the change of the valve position.

As mentioned before other systems may be used to pass form a configuration to the other; for instance manually changeable connections in the arterial line to the downstream position of the blood access and in the venous line to an upstream position of the blood access.

Alternatively the lines may be designed to present first conduits connecting the arterial line to both the upstream and the downstream position of the blood access and second conduits connecting the venous line to both the upstream and the downstream position of the blood access. In order to operate the configuration, means for selectively closing one of the first conduits between the arterial line and the blood access and means for selectively closing one of the conduits between the venous line and the blood access can be provided. Such closing means can be manually operable valves or valves controlled by the blood treatment apparatus. Pinch valves, cam valves or clamps having portions active on respective tube portions can be used. As a further alternative flow distribution means can be used able of connecting the arterial line with the upstream position of the access point and the venous line with the downstream position of the access point, in a first state of said flow distribution means, and able to connect the arterial line with the downstream position of the access point and the venous line with the upstream position of the access point, in a second state of said flow distribution means.

Figures 5 and 6 are graphs of measured pre and post dialyzer conductivities. The horizontal axis represent the lapsed times and the vertical axis represent the measured conductivity in mS/cm. In figures 5,6 it is assumed to start with the venous and arterial lines in normal condition and to switch the lines into the reversed condition during the time interval T of change of the conductivity of the dialysis fluid. As already mentioned it is possible to execute the method according to the invention starting with the reversed condition.

For determining the fluid flow rate in the blood access, a gradient between the conductivity of the dialysis fluid (Ci) at the dialyzer inlet and the blood (Cb) is created (Fig. 5). Hereto the conductivity of the dialysis liquid is increased from the conventional value of 14 mS/cm (first dialysis liquid having conductivity which corresponds roughly to the conductivity of blood) to 16 mS/cm (second dialysis liquid). The difference may be of another magnitude and, as already mentioned, can also be created by reducing the conductivity of the dialysis fluid. The conductivity of the second liquid is at least 2mS/cm (2 milli-Siemens / centimeter) higher than the conductivity of the first liquid if the conductivity of the first liquid is less or equal to 15mS/cm.

The conductivity gradient is preferably obtained by changing the sodium chloride concentration, but may also be obtained by varying the concentrations of any of the other electrolytes present in dialysis fluid. The change in electrolyte concentration can in advanced dialysis machines such as the Gambro AK 200 S® be executed by changing the settings or programming a step through the user interface. Use of conductivities instead of concentrations is simpler, more reliable, cheaper to implement as it employs the conventional sensors of the treatment apparatus, does not need determination of D or K in two different conditions.

In Figs. 5 and 6 the conductivity of the dialysis fluid Ci prepared by the dialysis monitor is increased from 14 to 16 mS/cm at time Ti. The conductivity Cn of the post dialyzer fluid, the effluent fluid, will begin to increase at time To with a delay To-Ti caused by the volume of the tubes and the dialyzer. Cn will reach a semi stable value only after some time. Because the increased conductivity of the dialysis liquid causes a transport of ions form the dialysis liquid to the blood, which therefore also slowly increases in conductivity, there will be a slow drift in of the post dialyzer conductivity. The value of Cn may be determined after the respective value has become substantially stable, as shown in Figure 5. In order to further improve the precision of the method the value of Cn may be extrapolated forward to the point in time of the flow reversal $T_{rev}$. Alternatively, the value of Cn may be determined while it is still increasing by estimating which substantially stable value Cn would have reached after an equilibrium has been established by using numerical methods such as curve fitting or and/or extrapolation, in order to determine the value of Cn at $T_{rev}$, shown in Figure 6. The latter approach will allow the method to be carried out in a shorter time span.

The next step is to reverse the flow at $T_{rev}$ (cf Figs. 5 and 6) as described above, i.e. a blood flow in a second direction is created in which the venous line 6 carries treated blood from the dialyzer 11 via arterial needle 5a to the upstream position of the blood access. The arterial line 5 draws in blood from the downstream position via venous needle 6a towards the dialyzer 11.

The effect of this measure is a further increase in the effluent conductivity, which after the flow reversal is referred to as Cr. Cr will reach a semi stable value only asymptotically. The value of Cr may be determined after it has become substantially stable, as shown in Figure 5. The value of Cr may be extrapolated backwards to the point in time of the flow reversal $T_{rev}$. Alternatively, the value may be determined while the conductivity is still increasing by estimating which substantially stable value Cr would have reached at $T_{rev}$ after an equilibrium has been established by using numerical methods such as curve fitting or extrapolation, as shown in figure 6.

The volumes in the dialyzer and connecting tubes that need to be exchanged cause the delay. During the delay period, changes in other parameters may occur and could influence the measurement negatively. The preferred method uses therefore the values extrapolated, to the point in time where the flow reversal took place. The above techniques allow estimating the value of Cn and of Cr at the same time Tr, thereby increasing the accuracy in Qa calculation.

Unit 85 may then calculate the fluid flow rate in the blood access in accordance with the formula:

$$Qa=(Tr-Quf)*(Cr-Ci)/(Cn-Cr),$$

wherein:

Qa =     fluid flow rate in the blood access

Tr =      transport rate of substances through the semipermeable membrane

Ci =      dialysis liquid conductivity upstream the treatment unit or dialyzer 11

Cn =     effluent conductivity referring to the dialysis liquid before flow reversal

Cr =      effluent conductivity referring to the dialysis liquid after flow reversal

Quf =    ultrafiltration flow rate (Quf).

The transport rate may be based on experience values of a particular dialyzer, such as the clearance, calculated from dialyzer capacity and flow rates or measured by comparing a predialysis blood sample with an initial dialysis liquid urea concentration. Alternatively the transport rate (Tr) corresponds to measured effective ionic dialysance (D) or to measured clearance K of the dialyzer, preferably the urea clearance value. The ultrafiltration flow rate Quf is on conventional dialysis machines continuously measured and monitored. The equation can therefore be solved and the fluid flow rate in the blood access is determined.

Alternatively to what described above with reference to figures 5,6, the measurement of Qa may be obtained by first configuring the lines in the reversed configuration. Then a change in conductivity or concentration (for instance by means of a step increase or decrease in the concentration of defined solutes in the dialysis liquid) is created and finally the concentration or conductivity of the dialysis liquid downstream the dialyzer is measured both for the liquid in reversed condition and for the liquid in normal condition. This second approach is convenient if the Qa measurement is carried out at the beginning of the dialysis session. Indeed the patient can be first connected to the treatment apparatus with the lines in reversed configuration; then when necessary the lines are reversed, the Qa calculated and the treatment can prosecute normally at high efficiency with no need of further line switching as the line are already in normal configuration.

In case the method is performed starting from the reversed configuration, then the Qa is still calculated as a function of the above-identified parameters.

If Tr is determined from the measured clearance K or the measured effective ionic dialysance D in vivo values obtained when said venous and arterial lines are in the normal configuration, the fluid flow rate (Qa) in said blood access is calculated by the formula Qa=(Tr-Quf)*(Cr-Ci)/(Cn-Cr), where Tr is the transport rate when the lines are in the normal configuration.

If Tr is obtained from the measured clearance K or the measured effective ionic dialysance D in vivo values obtained when said venous and arterial lines are in the reversed configuration, the fluid flow rate (Qa) in said blood access is calculated by the formula Qa=(Tr$_r$-Quf)*(C$_r$-Ci)/(C$_n$-C$_r$)+Tr$_r$, where Tr$_r$ is the transport rate when the lines are in the reversed configuration.

The measured clearance K or the measured effective ionic dialysance D in vivo values can obtained by the following steps:

a. passing a third dialysis liquid through the second chamber of said treatment unit, said dialysis liquid presenting a concentration for at least one substance, then

b. obtaining a third post treatment unit conductivity of the dialysis liquid or third post treatment unit concentration of said substance for the third dialysis liquid,

c. at least for a second time interval , increasing or decreasing the concentration of the substance in the third dialysis liquid for passing a fourth liquid through the second chamber inlet, said fourth liquid having a concentration of at least said substance different from the concentration of the same substance in the third liquid,

d. obtaining a fourth post treatment unit conductivity of the dialysis liquid or fourth post treatment unit concentration of said substance for the fourth dialysis liquid, calculating the in vivo value of K or D as a function of said third post treatment unit concentration or conductivity and of said fourth post treatment unit concentration or conductivity.

In particular the measured clearance K or the measured ionic dialysance D can be determined during the time interval T so as to use the change in conductivity necessary for the implementation of the present invention. In this case a separate modification of the liquid arriving at the second chamber 13 is not necessary and the third liquid corresponds to the first liquid (before the step in figures 5,6) and the fourth liquid corresponds to the second liquid (after the step in figures 5,6).

Practically if only ions concentration is altered, and again referring to the example of figure 5, Tr=K

$$K = (D+U) \cdot (1 - \frac{\Delta C_o}{\Delta C_i}) \qquad \frac{\Delta C_o}{\Delta C_i}$$ being the inverse of the rate between the step in conductivity of the dialysis fluid at the dialyser inlet and the corresponding step of the dialysis liquid at the outlet of the dialyzer

$$A = (K-U) \cdot (\frac{C_i - C_r}{C_r - C_n})$$

Another apparatus for determining the fluid flow rate in a blood access corresponding to the above disclosed process is as follows:

The apparatus is a blood treatment apparatus for determining the fluid flow rate (Qa) in a blood access having a downstream position and an upstream position, the apparatus comprising: a dialysis liquid source,

a. a treatment unit (11), having a semi permeable membrane (14) delimiting a first chamber (12) through which blood removed from said blood access passes and a second chamber(13) through which dialysis liquid passes,

b. a dialysis liquid line for circulating dialysis liquid in the second chamber (13);

c. an arterial line (5) connected to an inlet of the first chamber (12),

d. a venous line (6) connected to an outlet of the first chamber (12),

e. said arterial and venous lines (5, 6) being able to be configured according to at least a normal configuration, in which said arterial line (5) carries blood from said upstream position of said blood access, and said venous line (6) carries blood towards said downstream position of said blood access, and to at least a reversed configuration, in which said arterial line (5) carries blood from said downstream position of said blood access, and said venous line (6) carries blood towards said upstream position of said blood access,

f. means for switching (28) the venous and arterial lines (5, 6), during a time interval T, between one of said normal and reversed configurations to the other of said normal and reversed configurations,

g. means for varying (17, 18) a concentration (Ci) of at least a substance of the dialysis liquid upstream the treatment unit;

h. a sensor (20) operating downstream the treatment unit (11) for detecting a post-treatment unit conductivity of the dialysis liquid or a post treatment unit concentration of said substance in the dialysis liquid, and

i. a control unit (85) capable of performing the following steps:

o operating said varying means (17, 18) in such a way that, at least for said time interval T, said dialysis liquid circulating upstream the second chamber of the treatment unit (11) comprises at least a substance having a concentration (Ci) different from the concentration of the same substance in blood,

o obtaining from said sensor (20) a first post-treatment unit conductivity of the dialysis liquid or a first post treatment unit concentration of said substance in the dialysis liquid, for the venous and arterial lines (5, 6) being configured according to one of said normal or reversed configuration, said first conductivity or concentration relating to the dialysis liquid before switching the venous and arterial lines (5, 6) and during said time interval T;

o obtaining, from said sensor (20) a second post treatment unit conductivity of the dialysis liquid or post treatment unit concentration of said substance in the dialysis liquid, for the venous and arterial lines (5, 6) being configured according to the other of said normal or reversed configuration, said second conductivity or concentration relating to the dialysis liquid after switching of the venous and arterial lines and during said time interval T;

o calculating the fluid flow rate (Qa) in said blood access as a function of said first post treatment unit concentration or conductivity and of said second post treatment unit concentration or conductivity,

o acting on the varying means (17, 18) to keep substantially <u>constant</u> the concentration (Ci) of said at least a substance of the dialysis liquid upstream the treatment unit during said time interval T.

The above disclosed blood apparatus can present following additional features:

- said sensor comprises a post treatment unit conductivity (Cn,Cr) cell, or

- the varying means are designed for increasing or decreasing the concentration of one or more substances in the dialysis liquid, or

- during said time interval T the control unit (85) acts on said switching means to carry out the following consecutive sub-steps:

a. First configuring the said arterial and venous lines according to the normal configuration for obtaining said first concentration or first conductivity (Cn), and then

b. configuring the arterial and venous lines according to the reversed configuration for obtaining said second concentration or conductivity (Cr),

returning the arterial and venous lines to the normal configuration for prosecution of the blood treatment.;

In this case, after having configured the arterial and venous lines according to the reversed configuration the control unit can be able to perform a further step of checking if the arterial and venous lines are in said first or in said reversed configuration:

- or during said time interval T the control unit (85) acts on said switching means to carry out the following consecutive sub-steps:

a. First, configuring the said arterial and venous lines according to the reversed configuration for obtaining said first post treatment unit concentration or conductivity (Cr), and then

b. configuring the arterial and venous lines according to the normal configuration for obtaining said second concentration or conductivity (Cn) and then prosecuting the blood treatment.

- or the control unit (85) is able to perform a step of checking if the arterial and venous lines are in said normal or in said reversed configuration.

- or the control unit (85) is able to perform the following steps:

a. determining the transport rate (Tr) of ions though the semi permeable membrane,

b. obtaining the first post treatment unit conductivity (Cn, Cr) relating to the dialysis liquid before switching the venous and arterial lines,

c. obtaining the second post treatment unit conductivity (Cr, Cn) relating to the dialysis liquid after switching the venous and arterial lines,

c. calculating the fluid flow rate (Qa) in said blood access as a function of said first and second post treatment unit conductivities and of said transport rate.

In this particular case, the fluid flow rate (Qa) is calculated from the values of said transport rate (Tr), said first post treatment unit conductivity (Cn,Cr), said second post treatment unit conductivity (Cr,Cn), and the conductivity of the dialysis liquid (Ci) upstream the treatment unit. Alternatively in this case, said first and second post treatment unit conductivities (Cn, Cr) are obtained by said sensor.

- in any case the apparatus can further comprise means acting on the dialysis line for causing an ultrafiltration flow rate (Quf), in that case, the fluid flow rate (Qa) in said blood access is calculated by the formula $Qa=(Tr)*(Cr-Ci)/(Cn-Cr)$ or by the formula $Qa=(Tr-Quf)*(Cr-Ci)/(Cn-Cr)$, wherein Tr is determined from the measured clearance K or the measured ionic dialysance D in vivo values obtained when said venous and arterial lines are in the normal configuration. Alternatively or additionally in that case, $Tr_r$ is the transport rate obtained from the measured clearance K or the measured ionic dialysance D in vivo values obtained when said venous and arterial lines are in the reversed configuration, the fluid flow rate (Qa) in said blood access is calculated by the formula $Qa=(Tr_r-Quf)*(Cr-Ci)/(Cn-Cr)+Tr_r$.

- in any case the apparatus can further comprise means for preparing dialysis liquid with a conductivity different from said blood, preferably said means comprises means for controlled mixing of electrolyte concentrates with water.

- in any case the apparatus can have its switching means comprising:

  - manually changeable connections in the arterial line to the downstream position of the blood access and in the venous line to an upstream position of the blood access, or
  - first conduits connecting the arterial line to both the upstream and the downstream position of the blood access and second conduits connecting the venous line to both the upstream and the downstream position of the blood access, means for selectively closing one of the first conduits between the arterial line and the blood access and means for selectively closing one of the conduits between the venous line and the blood access, or
  - a valve able to connect the arterial line with the upstream position of the access point and the venous line with the downstream position of the access point in a first position of said valve and able to connect the arterial line with the downstream position of the access point and the venous line with the upstream position of the access point in a second position of said valve, or
  - flow distribution means for connecting the arterial line with the upstream position of the access point and the venous line with the downstream position of the access point in a first state of said flow distribution means and able to connect the arterial line with the downstream position of the access point and the venous line with the upstream position of the access point in a second state of said flow distribution means.

- in any case the apparatus can have its said treatment unit (11) comprising one selected in the group comprising:

  i. A dialyzer;
  ii. An hemofilter;
  iii. A plasmafilter;
  iv. An hemodiafilter;
  v. An ultrafilter.

Also disclosed is a software product comprising instructions executable by a control unit (85) of a blood treatment apparatus, said software as executed on said control unit (85) rendering it able of performing a method for determining a fluid flow rate (Qa) in a blood access having an upstream position and a downstream position using said blood treatment apparatus, comprising the steps of:

- passing a dialysis liquid through the second chamber (13) of said treatment unit (11), at least for a time interval T, said dialysis liquid upstream the treatment unit (11) comprising at least one substance having a concentration (Ci) different from the concentration of the same substance in blood,
- obtaining, downstream the second chamber (13) of said treatment unit (11), a first post-treatment unit conductivity of the dialysis liquid or a first post treatment unit concentration of said substance in the dialysis liquid, said first conductivity or concentration (Cn, Cr) relating to the venous and arterial lines configured according to one of said normal or reversed configuration, said first post treatment unit conductivity or concentration referring to the dialysis liquid before switching the venous and arterial lines and during said time interval T,
- switching the venous and arterial lines, during said time interval T, between one of said normal and reversed configurations to the other of said normal and reversed configurations,
- obtaining, downstream the second chamber (13) of said treatment unit (11), a second post treatment unit conductivity of the dialysis liquid or post treatment unit concentration of said substance in the dialysis liquid, said second conductivity or concentration (Cn, Cr) relating to the venous and arterial lines configured according to the other of said normal or reversed configuration, said second post treatment unit conductivity or concentration referring to the dialysis liquid after switching of the venous and arterial lines and during said time interval T;
- calculating the fluid flow rate (Qa) in said blood access as a function of

o said first post treatment unit concentration or conductivity and of

o said second post treatment unit concentration or conductivity,

and wherein during said time interval T of the passage of one dialysis liquid, the concentration Ci of said at least a substance in the liquid at the treatment unit inlet is kept substantially constant.

Said software as executed on said control unit (85) can

1) either render it able of performing said method wherein a step of checking if the arterial and venous lines are in said normal or in said reversed configuration is provided for.

2) or render it able of performing said method wherein during said time interval T the following consecutive sub-steps are provided with:

a. First configuring the said arterial and venous lines according to the normal configuration for obtaining said first concentration or first conductivity (Cn), and then

b. configuring the arterial and venous lines according to the reversed configuration for obtaining said second concentration or conductivity (Cr).

In case 2), the software as executed on said control unit (85) can render it able of performing said method wherein after having configured the arterial and venous lines according to the reversed configuration a further step of checking if the arterial and venous lines are in said normal or in said reversed configuration is provided for.

In case 1) or in case 2) with the additional alternative, said software as executed on said control unit (85) can render it able of performing said method wherein the step of checking if the arterial and venous lines are in the normal or in the reversed configuration comprises the following steps:

c. Determining the in vivo value of a parameter selected in the group comprising:

i. Effective ionic dialysance D or
ii. Effective clearance K or
iii. a parameter proportional to effective ionic dialysance or
iv. a parameter proportional to effective clearance,

d. comparing the in vivo value of said parameter with a corresponding threshold value for determining if the venous and arterial lines are in said normal or in said reversed configuration.

According to the invention an apparatus is provided for checking if the arterial and venous lines are in said normal or in said reversed configuration is provided for. This check can be executed at any time during treatment. If the check is carried out after the lines switching it can serve to provide an alert signal in case the operator (manual switching) or the apparatus (automatic switching) failed to return the lines in the normal configuration. The step of checking if the arterial and venous lines are in the normal or in the reversed configuration comprises the following steps:

Determining the in vivo value of a parameter selected in the group comprising:

a. Effective ionic dialysance D or
b. Effective clearance K or
c. a parameter proportional to effective ionic dialysance or
d. a parameter proportional to effective clearance,

Comparing the in vivo value of said parameter with a corresponding threshold value for determining if the venous and arterial lines are in said normal or in said reversed configuration. In case effective ionic dialysance D is used, any known method for in vivo determination of D can be used, such as the one described in EP 658 352.

A simple way of determining D comprises the steps of passing a third dialysis liquid through the second chamber inlet of said treatment unit, said dialysis liquid presenting a concentration for at least one substance, then obtaining a third post treatment unit conductivity of the dialysis liquid or third post treatment unit concentration of said substance for the third dialysis liquid, at least for a second time interval , increasing or decreasing the concentration of the substance in the third dialysis liquid for passing a fourth liquid through the second chamber inlet, said fourth liquid having a concentration of at least said substance different from the concentration of the same substance in the third liquid,

obtaining a fourth post treatment unit conductivity of the dialysis liquid or fourth post treatment unit concentration of said substance for the fourth dialysis liquid, calculating the in vivo value of D as a function of said third post treatment unit concentration or conductivity and of said fourth post treatment unit concentration or conductivity.

Once obtained the effective ionic dialysance value D, than D can be compared with a threshold value, which can be a set value or a calculated value or a measured value.

In vivo determination of D can of course be carried out during the time interval T.

In case the step of checking if the arterial and venous lines are in said normal or in said reversed configuration is carried out during the time interval T, then the following alternative procedure can be used:

- Comparing said obtained first post-treatment unit conductivity of the dialysis liquid or first post treatment unit concentration of said substance in the dialysis liquid with said obtained second post treatment unit conductivity of the dialysis liquid or second post treatment unit concentration of said substance in the dialysis liquid,
- Determining if said conductivity or concentration are increasing after the switching step. Indeed as can be seen in figure 5, if the conductivity of blood I lower than that of the dialysis liquid, after switching into reversed condition, a sudden increase in conductivity of the dialysis liquid downstream the dialyzer is registered.

The upstream conductivity cell should preferably calibrated relative to the downstream conductivity cell 20 for improved accuracy. Preferably temperature compensated conductivity cells are used to improve the accuracy of the method.

The value for Ci may be determined by measuring the conductivity of the dialysis fluid before it enters the dialyzer. Alternatively the set value for the dialysis fluid conductivity may be used, since the actual conductivity will only differ marginally from the set value as dialysis monitors control the conductivity of the dialysis fluid very accurately.

**Claims**

1. A blood treatment apparatus including:

    a blood treatment unit (11) having a semi permeable membrane (14) delimiting a first chamber (12) through which blood removed from said blood access passes and a second chamber (13) through which dialysis liquid passes, an arterial line (5) connected to an inlet of the first chamber, and a venous line (6) connected to an outlet of the first chamber, said arterial and venous lines (5, 6) being able to be configured according to at least a normal configuration, in which said arterial line (5) carries blood from said upstream position of said blood access, and said venous line (6) carries blood towards said downstream position of said blood access, and to at least a reversed configuration, in which said arterial line (5) carries blood from said downstream position of said blood access, and said venous line (6) carries blood towards said upstream portion of said blood access,

**characterized in that** said blood treatment apparatus is configured for carrying out a method for checking the operating configuration of the arterial and venous lines (5, 6) by executing comprising the steps of:

    - passing a dialysis liquid trough the second chamber (13) of said treatment unit (11), at least for a time interval (T) said dialysis liquid upstream the treatment unit (11) having a concentration (Ci) of at least one substance different from the concentration of the same substance in blood,
    - determining the in vivo value of a parameter selected in the group comprising:

        a. effective ionic dialysance (D) or
        b. effective clearance (K) or
        c. a parameter proportional to effective ionic dialysance (D) or
        d. a parameter proportional to effective clearance (K),

    - comparing the in vivo value of said parameter with a corresponding threshold value for determining if the venous and arterial lines (5, 6) are in said normal or in said reversed configuration.

2. Apparatus according to claim 1, wherein said threshold value is a set value or a calculated value.

3. Apparatus according to claim 1, wherein the step of in vivo determination of said parameter is carried out during the time interval (T).

4. A blood treatment apparatus including:

    a blood treatment unit (11) having a semi permeable membrane (14) delimiting a first chamber (12) through which blood removed from said blood access passes and a second chamber (13) through which dialysis liquid passes, an arterial line (5) connected to an inlet of the first chamber, and a venous line (6) connected to an outlet of the first chamber, said arterial and venous lines (5, 6) being able to be configured according to at least a normal configuration, in which said arterial line (5) carries blood from said upstream position of said blood access, and said venous (6) line carries blood towards said downstream position of said blood access, and to at least a reversed configuration, in which said arterial line (5) carries blood from said downstream position of said blood access, and said venous line (6) carries blood towards said upstream portion of said blood access,

**characterized in that** said blood treatment apparatus is configured for carrying out a method for checking the operating configuration of the arterial and venous lines (5, 6) comprising the steps of:

- passing a dialysis liquid trough the second chamber (13) of said treatment unit (11), at least for a time interval (T) said dialysis liquid upstream the treatment unit (11) having a concentration (Ci) of at least one substance different from the concentration of the same substance in blood,
- obtaining, downstream the treatment unit (11), a first post-treatment unit conductivity of the dialysis liquid or a first post treatment unit concentration of said substance in the dialysis liquid, said first conductivity or concentration relating to the venous and arterial lines (5, 6) configured according to one of said normal or reversed configuration;
- switching said lines from one of said configurations to the other;
- obtaining, downstream the treatment unit (11), a second post treatment unit conductivity of the dialysis liquid or post treatment unit concentration of said substance in the dialysis liquid, said second conductivity or concentration relating to the venous and arterial lines configured according to the other of said normal or reversed configuration;
- comparing said obtained first post-treatment unit conductivity of the dialysis liquid with said obtained second post treatment unit conductivity of the dialysis liquid or comparing said first post treatment unit concentration of said substance in the dialysis liquid with said second post treatment unit concentration of said substance in the dialysis liquid,
- evaluating the operating configuration of said lines by determining if said conductivity or concentration are increasing or decreasing after the switching step.

5. A blood treatment apparatus according to anyone of the claims from 1 to 4 comprising a control unit (85) and a software, wherein said software as executed on said control unit (85) renders it able of performing said method for checking the operating configuration of the arterial and venous lines (5, 6).

**Patentansprüche**

1. Blutbehandlungsvorrichtung beinhaltend:

eine Blutbehandlungseinheit (11) mit einer semipermeablen Membran (14), die eine erste Kammer (12), durch die das vom Blutzugang entfernte Blut geführt wird, und eine zweite Kammer (13), durch die die Dialyseflüssigkeit geführt wird, abgrenzt,
eine arterielle Leitung (5), die mit einem Eingang der ersten Kammer verbunden ist, und
eine venöse Leitung (6), die mit einem Ausgang der ersten Kammer verbunden ist,
wobei die arteriellen und venösen Leitungen (5, 6) dazu geeignet sind, nach mindestens einer normalen Konfiguration, in der die arterielle Leitung (5) das Blut von der aufwärtsliegenden Position des Blutzugangs fördert und die venöse Leitung (6) das Blut zur abwärtsliegenden Position des Blutzugangs fördert, und nach mindestens einer umgekehrten Konfiguration, in der die arterielle Leitung (5) das Blut von der abwärtsliegenden Position des Blutzugangs fördert und die venöse Leitung (6) das Blut zur aufwärtsliegenden Position des Blutzugangs fördert, konfiguriert werden können,

**dadurch gekennzeichnet, dass** die Blutbehandlungsvorrichtung konfiguriert ist zur Durchführung eines Verfahrens zur Prüfung der Betriebskonfiguration der arteriellen und venösen Leitungen (5, 6), umfassend die Schritte:

- Führen einer Dialyseflüssigkeit durch die zweite Kammer (13) der Behandlungseinheit (11) mindestens für eine Zeitspanne (T), wobei die Dialyseflüssigkeit aufwärts von der Behandlungseinheit (11) eine Konzentration (Ci) mindestens einer Substanz besitzt, die von der Konzentration derselben Substanz im Blut unterschiedlich ist,
- Bestimmen des In-vivo-Wertes eines Parameters ausgewählt aus der Gruppe umfassend:

  a. effektive ionische Dialysanz (D) oder
  b. effektive Clearance (K) oder
  c. einen Parameter proportional zur effektiven ionischen Dialysanz (D) oder
  d. einen Parameter proportional zur effektiven Clearance (K),

- Vergleichen des In-vivo-Wertes des Parameters mit einem entsprechenden Schwellenwert, um zu bestimmen, ob die venösen und arteriellen Leitungen (5, 6) sich in der normalen oder in der umgekehrten Konfiguration befinden.

2. Vorrichtung nach Anspruch 1, wobei es sich beim Schwellenwert um einen eingestellten Wert oder einen berechneten Wert handelt.

**3.** Vorrichtung nach Anspruch 1, wobei der Schritt der In-vivo-Bestimmung des Parameters während der Zeitspanne (T) durchgeführt wird.

**4.** Blutbehandlungsvorrichtung umfassend:

eine Blutbehandlungseinheit (11) mit einer semipermeablen Membran (14), die eine erste Kammer (12), durch die das vom Blutzugang entfernte Blut geführt wird, und eine zweite Kammer (13), durch die die Dialyseflüssigkeit geführt wird, abgrenzt,
eine arterielle Leitung (5), die mit einem Eingang der ersten Kammer verbunden ist, und
eine venöse Leitung (6), die mit einem Ausgang der ersten Kammer verbunden ist,
wobei die arteriellen und venösen Leitungen (5, 6) dazu geeignet sind, nach mindestens einer normalen Konfiguration, in der die arterielle Leitung (5) das Blut von der aufwärtsliegenden Position des Blutzugangs fördert und die venöse Leitung (6) das Blut zur abwärtsliegenden Position des Blutzugangs fördert, und nach mindestens einer umgekehrten Konfiguration, in der die arterielle Leitung (5) das Blut von der abwärtsliegenden Position des Blutzugangs fördert und die venöse Leitung (6) das Blut zur aufwärtsliegenden Position des Blutzugangs fördert, konfiguriert werden können,

**dadurch gekennzeichnet, dass** die Blutbehandlungsvorrichtung konfiguriert ist zur Durchführung eines Verfahrens zur Prüfung der Betriebskonfiguration der arteriellen und venösen Leitungen (5, 6), umfassend die Schritte:

- Führen einer Dialyseflüssigkeit durch die zweite Kammer (13) der Behandlungseinheit (11) mindestens für eine Zeitspanne (T), wobei die Dialyseflüssigkeit aufwärts von der Behandlungseinheit (11) eine Konzentration (Ci) mindestens einer Substanz besitzt, die von der Konzentration derselben Substanz im Blut unterschiedlich ist,
- Erhalten, abwärts von der Behandlungseinheit (11), einer ersten Leitfähigkeit der Dialyseflüssigkeit nach der Behandlungseinheit oder einer ersten Konzentration der Substanz in der Dialyseflüssigkeit nach der Behandlungseinheit , wobei die erste Leitfähigkeit oder Konzentration die venösen und arteriellen Leitungen (5, 6), die nach einer der normalen oder umgekehrten Konfiguration konfiguriert sind, betrifft;
- Umschalten der Leitungen von einer der Konfigurationen zur anderen;
- Erhalten, abwärts von der Behandlungseinheit (11), einer zweiten Leitfähigkeit der Dialyseflüssigkeit nach der Behandlungseinheit oder Konzentration der Substanz in der Dialyseflüssigkeit nach der Behandlungseinheit, wobei die zweite Leitfähigkeit oder Konzentration die venösen und arteriellen Leitungen, die konfiguriert sind nach der anderen der normalen oder umgekehrten Konfiguration, betrifft;
- Vergleichen der erhaltenen ersten Leitfähigkeit der Dialyseflüssigkeit nach der Behandlungseinheit mit der erhaltenen zweiten Leitfähigkeit der Dialyseflüssigkeit nach der Behandlungseinheit oder Vergleichen der ersten Konzentration der Substanz in der Dialyseflüssigkeit nach der Behandlungseinheit mit der zweiten Konzentration der Substanz in der Dialyseflüssigkeit nach der Behandlungseinheit,
- Bewerten der Betriebskonfiguration der Leitungen, durch bestimmen, ob die Leitfähigkeit oder Konzentration nach dem Umschaltschritt zu- oder abnimmt.

**5.** Blutbehandlungsvorrichtung nach einem der Ansprüche 1 bis 4, umfassend eine Steuereinheit (85) und eine Software, wobei die Software, wenn sie auf der Steuereinheit (85) ausgeführt wird, ihr ermöglicht, das Verfahren zur Prüfung der Betriebskonfiguration der arteriellen und venösen Leitungen (5, 6) durchzuführen.

**Revendications**

**1.** Appareil de traitement du sang comprenant:

une unité de traitement du sang (11) ayant une membrane semi-perméable (14) délimitant un premier compartiment (12) à travers lequel passe le sang déplacé dudit accès du sang, et un deuxième compartiment (13) à travers lequel passe le liquide de dialyse,
un conduit artériel (5) relié à une entrée du premier compartiment, et
un conduit veineux (6) relié à une sortie du premier compartiment,
lesdits conduits artériel et veineux (5, 6) pouvant être configurés selon au moins une configuration normale, dans laquelle ledit conduit artériel (5) porte le sang de ladite position en amont dudit accès du sang, et ledit conduit veineux (6) porte le sang vers ladite position en aval dudit accès du sang, et selon au moins une configuration inverse, dans laquelle ledit conduit artériel (5) porte le sang de ladite position en aval dudit accès du sang, et ledit conduit veineux (6) porte le sang vers ladite position en amont dudit accès du sang,

**caractérisé en ce que** ledit appareil de traitement du sang est configuré pour exécuter un procédé de vérification de la configuration opérationnelle des conduits artériel et veineux (5, 6) comprenant les étapes suivantes:

- faire passer un liquide de dialyse à travers le deuxième compartiment (13) de ladite unité de traitement (11) au moins pour un intervalle de temps (T), ledit liquide de dialyse en amont de l'unité de traitement (11) ayant une concentration (Ci) d'au moins une substance différente de la concentration de la même substance dans le sang,
- déterminer la valeur in vivo d'un paramètre choisi dans le groupe comprenant:

    a. dialysance ionique effective (D) ou
    b. clearance effective (K) ou
    c. un paramètre proportionnel à la dialysance ionique effective (D) ou
    d. un paramètre proportionnel à la clearance effective (K),

- comparer la valeur in vivo dudit paramètre avec une valeur de seuil correspondante pour déterminer si les conduits veineux et artériel (5, 6) sont dans ladite configuration normale ou dans ladite configuration inverse.

2. Appareil selon la revendication 1, où ladite valeur de seuil est une valeur configurée ou une valeur calculée.

3. Appareil selon la revendication 1, où l'étape de détermination in vivo dudit paramètre est exécutée pendant l'intervalle de temps (T).

4. Appareil de traitement du sang comprenant:

une unité de traitement du sang (11) ayant une membrane semi-perméable (14) délimitant un premier compartiment (12) à travers lequel passe le sang déplacé dudit accès du sang, et un deuxième compartiment (13) à travers lequel passe le liquide de dialyse,
un conduit artériel (5) relié à une entrée du premier compartiment, et
un conduit veineux (6) relié à une sortie du premier compartiment,
lesdits conduits artériel et veineux (5, 6) pouvant être configurés selon au moins une configuration normale, dans laquelle ledit conduit artériel (5) porte le sang de ladite position en amont dudit accès du sang, et ledit conduit veineux (6) porte le sang vers ladite position en aval dudit accès du sang, et selon au moins une configuration inverse, dans laquelle ledit conduit artériel (5) porte le sang de ladite position en aval dudit accès du sang, et ledit conduit veineux (6) porte le sang vers ladite position en amont dudit accès du sang,

**caractérisé en ce que** ledit appareil de traitement du sang est configuré pour exécuter un procédé de vérification de la configuration opérationnelle des conduits artériel et veineux (5, 6) comprenant les étapes suivantes:

- faire passer un liquide de dialyse à travers le deuxième compartiment (13) de ladite unité de traitement (11) au moins pour un intervalle de temps (T), ledit liquide de dialyse en amont de l'unité de traitement (11) ayant une concentration (Ci) d'au moins une substance différente de la concentration de la même substance dans le sang,
- obtenir en aval de l'unité de traitement (11) une première conductivité du liquide de dialyse dans l'unité de post-traitement ou une première concentration de ladite substance dans le liquide de dialyse dans l'unité de post-traitement, ladite première conductivité ou concentration concernant les conduits veineux ou artériel (5, 6) configurés selon l'une entre ladite configuration normale ou inverse;
- faire passer lesdits conduits de l'une desdites configurations à l'autre;
- obtenir en aval de l'unité de traitement (11) une deuxième conductivité du liquide de dialyse dans l'unité de post-traitement ou concentration de ladite substance dans le liquide de dialyse dans l'unité de post-traitement, ladite deuxième conductivité ou concentration concernant les conduits veineux ou artériel configurés selon l'autre entre ladite configuration normale ou inverse;
- comparer ladite première conductivité du liquide de dialyse dans l'unité de post-traitement obtenue avec ladite deuxième conductivité du liquide de dialyse dans l'unité de post-traitement ou comparer ladite première concentration de ladite substance dans le liquide de dialyse dans l'unité de post-traitement avec ladite deuxième concentration de ladite substance dans le liquide de dialyse dans l'unité de post-traitement,
- évaluer la configuration opérationnelle desdits conduits en déterminant si ladite conductivité ou concentration est en augmentation ou en diminution après l'étape de passage.

5. Appareil de traitement du sang selon une quelconque des revendications 1 à 4, comprenant une unité de commande (85) et un logiciel, où ledit logiciel exécuté sur ladite unité de commande (85) permet à cette dernière d'exécuter ledit procédé de vérification de la configuration opérationnelle des conduits artériel et veineux (5, 6).

Fig.1

Fig.2

Fig. 3

Fig. 4

EP 1 938 847 B1

Fig. 5

17

*Fig.6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5685989 A
- US 5595182 A
- US 5453576 A
- US 5510716 A
- US 5510717 A
- US 5312550 A
- EP 928614 A
- WO 0024440 A
- US 5605630 A
- US 5894011 A
- EP 658352 A